# EUROPEAN PATENT APPLICATION

(11) **EP 4 497 749 A1**
(43) Date of publication of application: **29.01.2025**
(21) Application number: 23775268.8
(22) Date of filing: 21.03.2023
(51) Int. Cl.: C07D 317/36

(54) **SYNTHESIS METHOD FOR ALKYLENE CARBONATE**

(30) Priority: 22.03.2022 KR 20220035182
(71) Applicant: Seoul National University R&DB Foundation, Seoul 08826 (KR)
(72) Inventor: NAM, Ki-Tae, Seoul 06102 (KR); JANG, Jun-Ho, Seoul 08814 (KR)
(74) Representative: Maiwald GmbH
(86) International application number: PCT/KR2023/003720
(87) International publication number: WO 2023/182772

(57) **Abstract**

The present invention relates to a synthesis method for alkylene carbonate, for example, alkylene carbonate comprising propylene carbonate, ethylene carbonate, and the like and, more specifically to, a synthesis method for alkylene carbonate comprising mixing a liquid compound of chemical formula 1 and hydrogen carbonate (MHCO₃). According to the synthesis method of the present invention, use of carbon dioxide, ethylene oxide, propylene oxide, and the like is unnecessary, and alkylene carbonate may be obtained in high yield even under relatively low-temperature atmospheric pressure.

## Description

### Technical Field

The present disclosure relates to a method for synthesizing alkylene carbonate, more specifically, to a method for synthesizing alkylene carbonate for being converted to alkylene carbonate such as ethylene carbonate and propylene carbonate with high yield and concentration without addition of additional solvents, catalysts, and carbon dioxide, under conditions in which ethylene oxide, propylene oxide, and the like, are not used.

### Background Art

Ethylene carbonate and propylene carbonate are not only used as raw materials for polycarbonate, pharmaceutical intermediates, oxyalkylation agents in a dye synthesis process, process equipment protectors, solvents in a process for producing textiles, and the like, but the scope of use thereof has been recently expanded, including as solvents for polymer electrolytes in secondary batteries.

The conventionally known synthesis of ethylene carbonate and propylene carbonate was undertaken by reacting carbon dioxide with ethylene oxide or propylene oxide in the presence of a catalyst, but in this case, in order for the accompanying reaction to achieve an industrially useful reaction rate, high temperature and high pressure carbon dioxide gas or liquid was required. In addition, in the case of ethylene oxide, there was a problem in that a large amount of carbon dioxide is released during the process of synthesizing ethylene oxide from ethylene. Since ethylene oxide or propylene oxide tends to decompose or polymerize under high temperature and pressure conditions, various catalysts have been developed to alleviate reaction conditions.

For example, Japanese Patent Laid-Open No. 9-67365 discloses a method of using Kl (potassium iodide) as a catalyst, and Japanese Patent Publication No. 59-13776 discloses a method of using a tetraalkyl phosphnium halide such as tributylmethyl phosphonium iodide as a catalyst, respectively. These patents state that a yield of 50 to 95% is obtained when reacted at a temperature of 100 to 170°C for 1 to 5 hours, but in order to obtain a high yield, there is a problem in that the reaction should be performed at a high temperature for a long period of time, and a content of moisture of carbon dioxide and alkylene oxide, which are raw materials, should be controlled to be below several hundred ppm. Accordingly, a method using an ion exchange resin as a catalyst has been proposed, but this method has the problem of a low yield at 80 to 100°C.

In summary, the conventional technology for commercially producing ethylene carbonate and propylene carbonate has difficult reaction conditions, including that high-pressure gas and liquid carbon dioxide should be reacted at a high temperature, and the content of moisture of the raw material should be very low. In addition, despite the development of various catalysts, the generation of carbon dioxide in the process of producing ethylene oxide cannot be avoided.

Accordingly, when a technology that does not require the use of high-pressure carbon oxide, solvents, and catalysts, and does not require the use of ethylene oxide or propylene oxide, and can obtain ethylene carbonate and propylene carbonate in high concentrations, is developed, it is expected that the technology will be widely applied in the related fields.

### Summary of Invention

### Technical Problem

An aspect of the present disclosure is to provide a method for synthesizing alkylene carbonate not requiring the use of ethylene oxide and propylene oxide, carbon oxide, solvents, catalysts, and the like.

### Solution to Problem

Accordingly, according to an aspect of the present disclosure, a method for synthesizing alkylene carbonate is provided, the method including an operation of mixing a compound represented by the following Formula 1, which is a liquid, with hydrogen carbonate (MHCO₃).

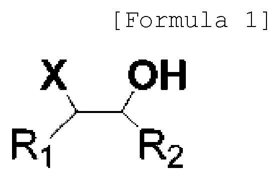

In Formula 1, X is halogen selected from the group consisting of fluorine, chlorine, bromine, and iodine, and R₁ and R₂ are independently selected from the group consisting of hydrogen and (C₁-C₃) alkyl groups.

### Advantageous Effects of Invention

According to the present disclosure, in the method for synthesizing alkylene carbonate, the use or further addition of ethylene oxide, propylene oxide, or carbon dioxide is not required, alkylene carbonate may be obtained in a high yield, even under relatively low temperature and low pressure conditions, and since the use of solvents may be eliminated, a subsequent purification process may be simple, which may be preferable in terms of process economy.

### Brief description of drawings

FIG. 1 is a graph illustrating a change in products depending on temperature when synthesizing ethylene carbonate according to the present disclosure.
FIG. 2 illustrates results of observing a change in reactants and products over time using NMR when synthesizing ethylene carbonate according to the present disclosure.
FIG. 3 graphically illustrates a yield of ethylene carbonate according to the type of halogen.
FIG. 4 illustrates NMR data of a product in a reaction solution after reaction in an example of the present disclosure.
FIG. 5 is a graph illustrating a change in products over temperature and time when synthesizing ethylene carbonate according to the present disclosure.
FIG. 6 graphically illustrates results of confirming a production yield of carbonate under conditions without using a solvent.

### Best Mode for Invention

Hereinafter, preferred embodiments of the present disclosure will be described with reference to the attached drawings. However, the embodiments of the present disclosure may be modified to have various other forms, and the scope of the present disclosure is not limited to the embodiments described below.

The method for synthesizing alkylene carbonate including ethylene carbonate, propylene carbonate, and the like, according to the present disclosure, does not require the use of ethylene oxide, propylene oxide, carbon dioxide, and the like, and alkylene carbonate may be obtained in a high yield, even under relatively low temperature and pressure conditions, and since the use of solvents may be eliminated, a subsequent purification process may be simple, which is preferable in terms of process economy.

In more detail, the method for synthesizing alkylene carbonate of the present disclosure includes an operation of mixing a compound represented by the following Formula 1, which is a liquid, with hydrogen carbonate (MHCO₃).

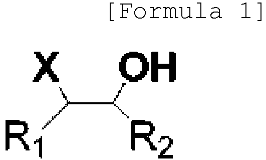

In this case, in Formula 1, X is halogen selected from the group consisting of fluorine, chlorine, bromine, and iodine, and R₁ and R₂ are independently selected from the group consisting of hydrogen and (C₁-C₃) alkyl groups, respectively. Preferably, R₁ and R₂ are respectively independently a hydrogen or methyl group.

In a compound of Formula 1, the positions of X and - OH groups can be interchanged, and the positions of R₁ and R₂ can also be interchanged.

More specifically, the compound of Formula 1 may be halogenated ethanol of Formula 1-1 below, or halogenated propanol of Formula 1-2 or Formula 1-3 below.

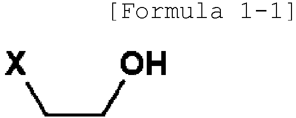

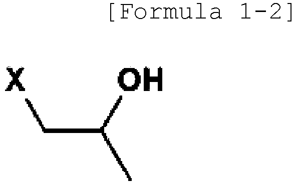

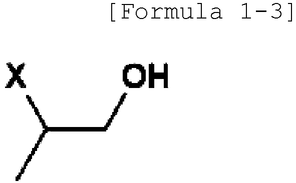

Meanwhile, the hydrogen carbonate (MHCO₃) may be in liquid or solid form. In more detail, the method for synthesizing alkylene carbonate of the present disclosure employs a compound of Formula 1, which is a liquid, as a reactant, for example, halogenated alcohol in liquid form and hydrogen carbonate (MHCO₃) in solution or solid form, but does not require ethylene oxide, propylene oxide, additional carbon dioxide or solvents. The hydrogen carbonate (MHCO₃) is preferably in the form of solid powder.

By performing the mixing operation, liquid alkylene carbonate may be obtained according to the following Scheme 1. According to the present disclosure, since a solvent is not essential, a subsequent separation process of the solvent from the product is simple, and high-concentration propylene carbonate or ethylene carbonate may be obtained as the product in a single operation.

2M⁺ + 2HCO₃⁻ + 2 (compounds of Formula 1) - 2alkylene carbonate + 2MX + 2H₂O [Scheme 1]

As can be seen in Scheme 1, bicarbonate (HCO₃⁻) derived from hydrogen carbonate in solution or solid form reacts with compounds of Formula 1 in liquid form, such as halogenated alcohol. Among the reaction products, MX is obtained from solid form and thus can be easily separated by separation of the solid-liquid form thereof.

In Scheme 1, metal (M⁺) may be an alkali metal cation, for example, selected from the group consisting of Li, Na, K, and Cs. In addition, metal (M⁺) may be an organic nitrogen compound-based cation, and may be selected from the group consisting of, for example, ammonium (NH₄⁺), primary ammonium (NH₃⁺CH₂CH₂OH, NH₃⁺CH₂CH₃, NH₃⁺CH₂CH₂CH₃, and the like), secondary ammonium (NH₂⁺(CH₂CH₂OH)₂, NH₂⁺(CH₂CH₃)₂, NH₂⁺(CH₂CH₂CH₃)₂, and the like), tertiary ammonium (NH⁺(CH₂CH₂OH)₃, NH⁺(CH₂CH₃)₃, NH⁺(CH₂CH₂CH₃)₃, and the like), and quaternary ammonium (N⁺(CH₂CH₂OH)₄, N⁺(CH₂CH₃)₄, N⁺(CH₂CH₂CH₃)₄, and the like) .

In the present disclosure, the mixing operation may be performed at a temperature within a range of 40°C or higher and lower than 150°C, for example, at a temperature within a range of 45°C to 90°C, preferably at a temperature within a range of 60°C to 90°C, and more preferably at a temperature within a range of 65°C to 85°C. In the mixing operation, propylene carbonate and ethylene carbonate can be produced with a yield of 70% or more, and even 80% or more, without applying additional pressure.

On the other hand, in the operation of producing ethylene carbonate, when the temperature is below the range of the present disclosure, the reaction may be somewhat slow, and when the temperature exceeds the range of the present disclosure, there may be a problem in that a yield of alkylene carbonate is lowered due to an increased amount of ethylene glycol and propylene glycol generated due to the increase in side reactions as illustrated in the following Scheme 3.

HCO₃⁻ + a compound of Formula 1 = Glycol + CO₂ + X- [Scheme 3]

A time for which the mixing operation of the present disclosure is performed may be 15 minutes to 24 hours, for example, 30 minutes to 6 hours. When a reaction time is less than 15 minutes, a yield of a product may be insufficient, and when the reaction time exceeds 24 hours, an increase in the yield of the product due to an increase in reaction time is not significant, which is not preferable in terms of process economy.

However, in the present disclosure, the use of additional solvents in the mixing operation is not excluded, and if necessary, water; various types of polar organic solvents such as alcohol, dimethylformamide, acetone, ether, ester, and the like; and a mixture of at least two of these components can be used. Preferably, water can be mixed as a solvent.

The method for synthesizing propylene carbonate and ethylene carbonate of the present disclosure preferably uses a closed reactor. In this case, external pressure of a reaction vessel is maintained at atmospheric pressure, and internal pressure of the closed reaction vessel may change as the reaction progresses. In addition, when the reaction vessel is not sealed, the reaction can proceed even if both the internal and external pressures are maintained at atmospheric pressure.

Meanwhile, in the mixing operation, a compound of Formula 1, for example, halogenated alcohol and hydrogen carbonate (MHCO₃) are mixed in a molar ratio of 1:0.5 to 2, preferably in the same molar ratio of 1:1.

Compounds of Formula 1 in liquid form, which are reactants of the present disclosure, such as halogenated alcohol and hydrogen carbonate (MHCO₃) in solution or solid form, can be obtained through synthesis or commercially obtained, respectively, and the acquisition routes and synthesis processes thereof are not particularly limited.

For example, the compound represented by Formula 1 may be obtained by mixing a halogen-substituted nitrogen compound with alkylene in a solvent, according to the following Scheme 2 below.

[Scheme 2] 2alkylene + 2XY + 2H₂O = 2 compound of Formula 1+ 2HY

In Scheme 2, X is halogen selected from the group consisting of fluorine, chlorine, bromine, and iodine, Y is a nitrogen compound and alkylene may be selected from the group consisting of ethylene and propylene.

In this case, the nitrogen compound (Y) is preferably a nitrogen compound of which conjugate acid (HY) of the nitrogen compound (Y) has a pKa of 7 to 14, preferably a pKa of 7 to 11, for example, is preferably at least one selected from a compound having an imide functional group and ammonium compounds, and more specifically, as the compound having an imide function group, succinimide, hydantoin, cyanurate, and the like may be used, and as the ammonium compound, ammonium, monoethanol ammonium, diethanol ammonium, and the like may be used.

According to the method for synthesizing alkylene carbonate of the present disclosure, alkylene carbonate in liquid form can be obtained in a high yield and concentration, so a subsequent purification process is not required. However, in some cases, a purification operation may be performed to improve purity, for example, a drying operation may be additionally performed.

As described above, according to the present disclosure, the use of high temperature, high pressure, catalyst, solvent, ethylene oxide, propylene oxide, and carbon dioxide is not necessarily required, and the purification process may be performed in a single operation at mild temperature and pressure. In addition, alkylene carbonate can be obtained in a high yield and no further subsequent purification is required.

Hereinafter, the present disclosure will be described in more detail through specific examples. The following examples are merely examples to aid understanding of the present disclosure, and the scope of the present disclosure is not limited thereto.

### Mode for Invention

### Example

### 1. Synthesis of alkylene carbonate

### Manufacturing Example 1

5 mmol of each of liquid bromoethanol and NaHCO₃ powder were mixed with 10 ml of water in a hydrothermal synthesis reaction vessel with a capacity of 11 ml, and then sealed under normal pressure conditions and heated at a temperature within a range of 25 to 105°C for 45 minutes. Thereafter, a product present in a reaction solution was observed at temperatures of 25, 45, 50, 65, 85, and 105°C.

### Manufacturing Example 2

1 mmol of each of liquid bromoethanol and NaHCO₃ powder were mixed with 10 ml of water in a vial with a capacity of 20 ml, and then reacted at 50°C for 8 hours under normal pressure conditions, and then a product present in a reaction solution was observed.

### Manufacturing Example 3

9.57 g and 6.43 g of each of liquid bromoethanol and NaHCO₃ powder were mixed in a hydrothermal synthesis reaction vessel with a capacity of 11ml, and then sealed under normal pressure and heated at temperatures of 45°C, 65°C, and 85°C for 12 hours, and then a product present in a reaction solution was observed.

### Manufacturing Example 4

Reaction was performed under the same conditions as in Manufacturing Example 2, except that liquid iodoethanol was used instead of liquid bromoethanol, and then a product present in a reaction solution was observed.

### 2. Confirmation of Product

### (1) Change in product depending on temperature

When alkylene carbonate was synthesized by the same process as in Manufacturing Example 1, the product present in the reaction solution was observed through NMR analysis at temperatures of 25, 45, 65, 85, and 105°C.

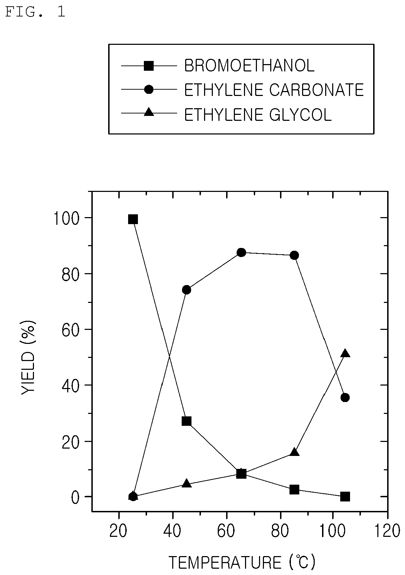

FIG. 1 illustrates the results as a graph. As can be seen in FIG. 1, according to the method of the present disclosure, it was confirmed that alkylene carbonate may be obtained with a yield of 75% or more at a mild temperature within a range of 45 to 90°C at normal pressure, and alkylene carbonate may be obtained with a yield of about 85% or more at a temperature within a range of 60 to 85°C.

### (2) Change in product over time

When synthesizing alkylene carbonate by the same process as in Manufacturing Example 2, the changing aspect of HCO₃⁻ into ethylene carbonate was observed by NMR during reaction times of 0 h, 2 h, 4 h, and 6 h while heating at 50°C.

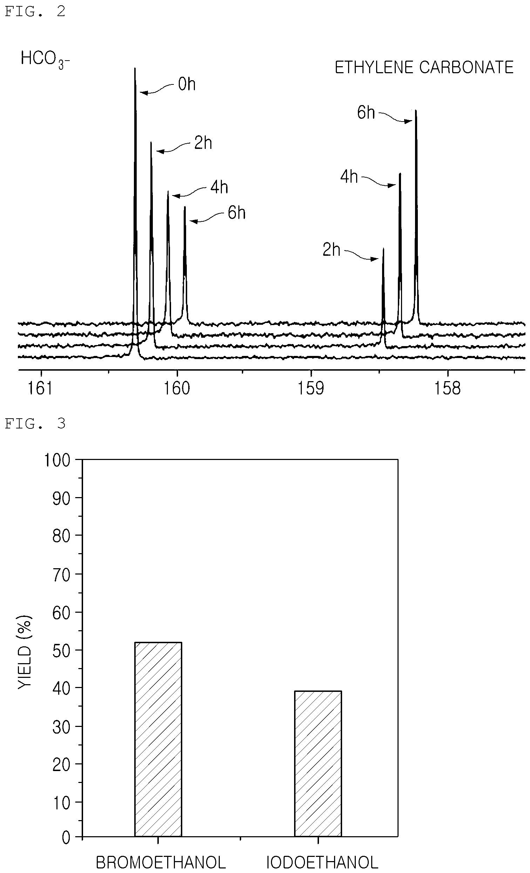

FIG. 2 illustrates these results, and it was confirmed that ethylene carbonate, which did not exist at the start of the reaction (0h), was produced and the product increased over time. In addition, according to the same results, it was confirmed that bromoethanol may be converted to ethylene carbonate even when a reaction vessel is not sealed and both inside and outside the container are maintained at atmospheric pressure.

### (3) Change in product depending on halogen type

When synthesizing alkylene carbonate by the same process as in Manufacturing Example 2 and Manufacturing Example 4, the product present in the reaction solution was observed by NMR. As a result, as shown in FIG. 3, it was confirmed that ethylene carbonate was obtained in both Manufacturing Example 2 using bromoethanol and Manufacturing Example 5 using iodoethanol.

### (4) Change in product over temperature and time

When synthesizing alkylene carbonate by the same process as in Manufacturing Example 1, the product present in the reaction solution was observed by NMR at 45, 55, 65, 75, and 85°C over the reaction times.

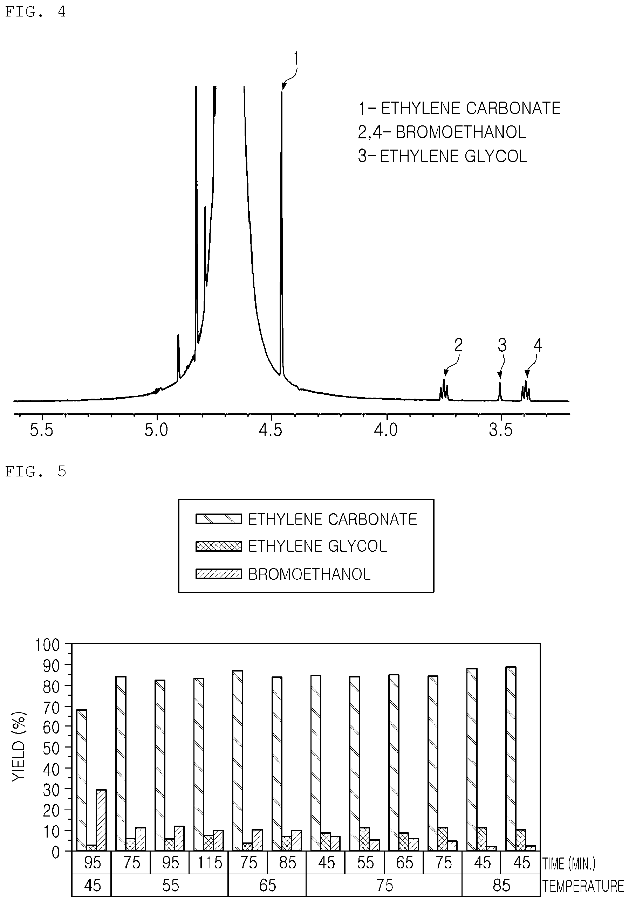

Thereamong, the reaction solution after heating at a temperature of 45°C for 95 hours was measured by NMR to confirm the results shown in FIG. 4, and it was also confirmed that a high yield of more than 80% could be obtained even under various temperature and time conditions as shown in FIG. 5.

### (4) Observation of carbonate production under conditions without using a solvent

When synthesizing alkylene carbonate by the same process as in Manufacturing Example 3, the product present in the reaction solution was observed by NMR depending on the reaction temperature after heating is performed at 45, 65, and 85°C for 12 hours.

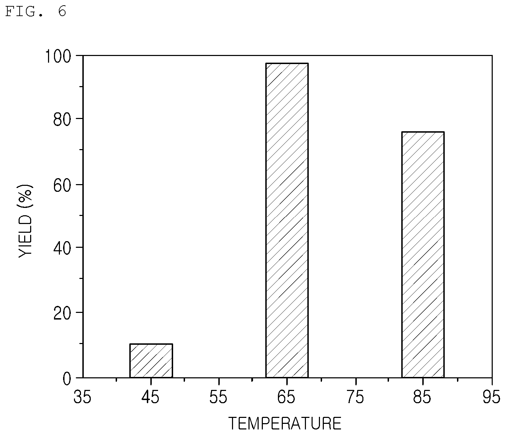

Thereamong, after heating is performed at 65°C for 12 hours, a carbonate yield reached 97% as shown in FIG. 6, and it was confirmed that ethylene carbonate was produced in a high yield using only a reactant even under conditions without using a solvent.

While example embodiments have been illustrated and described above, it will be apparent to those skilled in the art that modifications and variations could be made without departing from the scope of the present disclosure as defined by the appended claims.

## Claims

1. A method for synthesizing alkylene carbonate, comprising:
mixing a compound represented by the following Formula 1, which is a liquid, with hydrogen carbonate (MHCO₃), 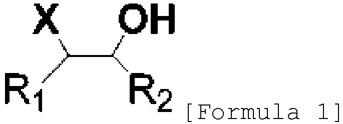 in Formula 1,
X is a halogen selected from the group consisting of fluorine, chlorine, bromine, and iodine, and
R₁ and R₂ are independently selected from the group consisting of hydrogen and (C₁-C₃) alkyl groups.

2. The method for synthesizing alkylene carbonate of claim 1, wherein the hydrogen carbonate (MHCO₃) is liquid or solid form.

3. The method for synthesizing alkylene carbonate of claim 1, wherein, in the mixing operation, liquid alkylene carbonate is obtained according to the following Scheme 1,
2M⁺ + 2HCO₃⁻ + 2 (compounds of Formula 1) - 2alkylene carbonate + 2MX + 2H₂O [Scheme 1]

4. The method for synthesizing alkylene carbonate of claim 2, wherein, in Scheme 1, metal (M⁺) is selected from the group consisting of an alkali metal cation, ammonium (NH₄⁺), primary ammonium, secondary ammonium, tertiary ammonium, and quaternary ammonium.

5. The method for synthesizing alkylene carbonate of claim 1, wherein the compound represented by Formula 1 is halogenated ethanol.

6. The method for synthesizing alkylene carbonate of claim 1, wherein the mixing operation is performed at a temperature within a range of 40°C or higher and lower than 150°C.

7. The method for synthesizing alkylene carbonate of claim 1, wherein, in the mixing operation, the compound of Formula 1 and hydrogen carbonate (MHCO₃) are mixed in a molar ratio of 1:0.5 to 2.

8. The method for synthesizing alkylene carbonate of claim 1, wherein the mixing operation is performed under atmospheric pressure conditions.

9. The method for synthesizing alkylene carbonate of claim 1, wherein the compound represented by Formula 1 is obtained according to the following Scheme 2, by mixing a halogen-substituted nitrogen compound with alkylene in a solvent,
[Scheme 2] 2alkylene + 2XY + 2H₂O = 2 (compounds of Formula 1) + 2HY
in Scheme 2, X is halogen selected from the group consisting of fluorine, chlorine, bromine, and iodine, and
Y is a nitrogen compound, and alkylene is selected from the group consisting of ethylene and propylene.

10. The method for synthesizing alkylene carbonate of claim 9, wherein the nitrogen compound has a pKa of 7 to 14 of conjugate acid (HY) of the nitrogen compound.

11. The method for synthesizing alkylene carbonate of claim 9, wherein the nitrogen compound is selected from the group consisting of a compound having an imide functional group and an ammonium compound.

12. The method for synthesizing alkylene carbonate of claim 9, wherein the nitrogen compound is at least one selected from the group consisting of succinimide, hydantoin, cyanurate, and ammonium compounds.

13. The method for synthesizing alkylene carbonate of claim 1, wherein the mixing operation is performed without a solvent; or
the mixing operation is performed under water; at least one polar organic solvent selected from alcohol, dimethylformamide, acetone, ether, and ester; or a mixture thereof.

14. The method for synthesizing alkylene carbonate of claim 1, wherein the hydrogen carbonate ion (HCO₃⁻) is obtained by reacting at least one component selected from the group consisting of an inorganic base, secondary amine, tertiary amine, and quaternary ammonium hydroxide salts with carbon dioxide in the presence of water.
